(19) Europäisches Patentamt
European Patent Office
Office européen des brevets

(11) **EP 3 498 823 A1**

(12) **EUROPEAN PATENT APPLICATION**
published in accordance with Art. 153(4) EPC

(43) Date of publication:
**19.06.2019 Bulletin 2019/25**

(21) Application number: **17839584.4**

(22) Date of filing: **10.08.2017**

(51) Int Cl.:
*C12N 5/071* (2010.01)    *G01N 1/28* (2006.01)
*G01N 1/30* (2006.01)

(86) International application number:
**PCT/JP2017/029126**

(87) International publication number:
**WO 2018/030520 (15.02.2018 Gazette 2018/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.08.2016 JP 2016157873**

(71) Applicant: **Shiseido Company Ltd.**
**Chuo-ku**
**Tokyo 104-0061 (JP)**

(72) Inventors:
• **KAJIYA, Kentaro**
**Yokohama-shi**
**Kanagawa 224-8558 (JP)**
• **YAMASHITA, Toyonobu**
**Yokohama-shi**
**Kanagawa 224-8558 (JP)**

(74) Representative: **Kilger, Ute**
**Boehmert & Boehmert**
**Anwaltspartnerschaft mbB**
**Pettenkoferstrasse 22**
**80336 München (DE)**

(54) **TRANSPARENT SKIN SAMPLE**

(57)    The present invention address the problem of providing a transparent skin sample by removing epidermis via enzymatic treatment.

FIG. 2

(A)    (B)

EP 3 498 823 A1

## Description

FIELD

The present invention relates to a transparent skin sample that allows observation of subepidermal tissue under a light sheet microscope, to a method for its production, and to a method of treating skin sections.

BACKGROUND

As techniques for three-dimensional analysis of tissue, techniques using fluorescent microscopes have been conducted. However, when observing tissue under a fluorescent microscope, the tissue must be embedded and cut into thin slices with a microtome. While such methods have been suitable for three-dimensional analysis of microstructures such as cell structures, observation of larger tissue structures has been problematic in terms of sample preparation and fading. In order to solve these problems, light sheet microscopes have been developed in which sheet-like excitation light is irradiated from the sample side to obtain an optical cross-section, and they allow three-dimensional analysis of larger samples such as biological samples or biological tissue. Highly transparent samples such as zebrafish or medaka have been suitable for using light sheet microscopes, whereas opaque samples such as mammalian biological tissue or tissue sections have required clearing treatment.

In recent years, a great number of transparent techniques for tissue samples have been developed (PTL 1). Clearing techniques are largely classified as techniques using organic solvent clearing agents (NPL 1) techniques using water-soluble clearing agents (NPL 2), but these both have issues in terms of transparent difficulty, transparent sample refractive index, and maintenance of antigenicity. Moreover, it was known that when human tissue is subjected to a clearing treatment, it is difficult to clearing human tissue due to its abundant matrix and high light scattering properties.

CITATION LIST

PATENT LITERATURE

[PTL 1] Japanese Unexamined Patent Publication No. 2014-5231

NON-PATENT LITERATURE

[NPL 1] Cell (2014) vol. 159, Issue 4, pp. 896-910
[NPL 2] Nature Neuro Science (2015), vol.18, No.10, pp1518-1529
[NPL 3] Cell (2014) vol. 157, Issue 3, pp. 726-39
[NPL 4] Proceedings of the IEEE Conference on Computer Vision and Pattern Recognition Workshops:29-37

SUMMARY

TECHNICAL PROBLEM

During attempts for clearing human skin sections, the present inventors have discovered a problem in being unable to observe areas directly under the epidermis of cleared skin samples, in addition to difficulty of clearing due to the abundant matrix in skin.

SOLUTION TO PROBLEM

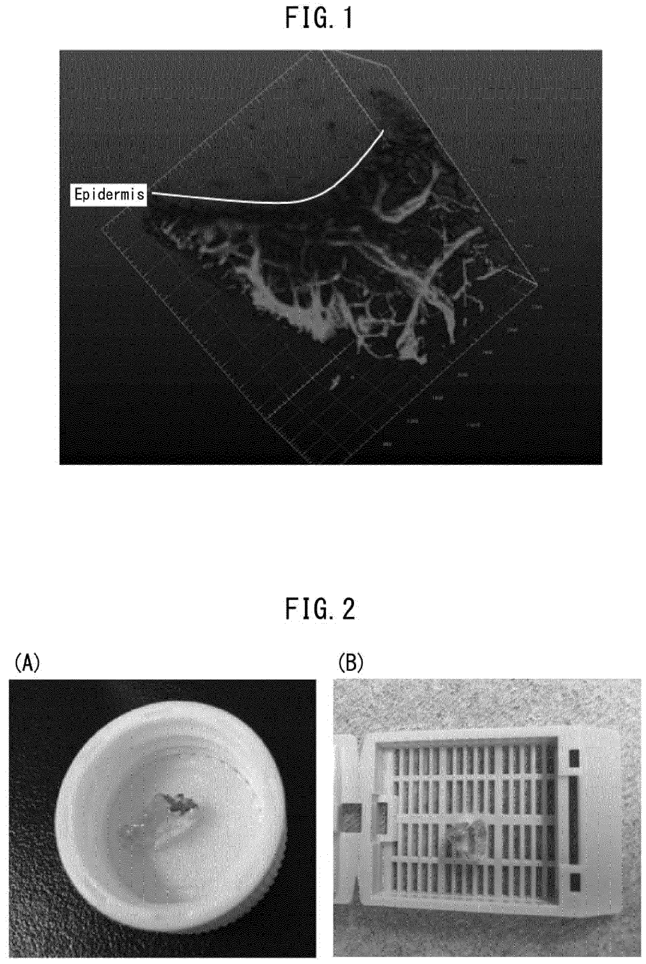

The present inventors have carried out diligent research with the aim of solving the aforementioned problem, and have found that clearing of epidermal sections is insufficient (Fig. 1). Upon examining methods for eliminating epidermal sections alone from skin sections, it has been found that the epidermal section can be removed through treatment with Dispase solution, thereby the capillary structure directly under the epidermis can be observed, and thus the present invention has been achieved.

Specifically, the present invention relates to a transparent skin sample wherein the epidermis is not accompanied with the sample and its antigenicity is maintained, and the transparent skin sample allowing observation of subepidermal tissue under a light sheet microscope.

According to another aspect, the invention also relates to a method for producing a transparent skin sample from an obtained skin section, and a transparent skin sample produced by the method.

According to yet another aspect, the invention also relates to a method of treating a skin section, and to a method of observing a skin sample treated by the treatment method under a light sheet microscope.

ADVANTAGEOUS EFFECTS OF INVENTION

By using the transparent skin sample of the invention it is possible to observe the structure directly under the epidermis.

BRIEF DESCRIPTION OF DRAWINGS

FIG. 1 is a three-dimensional image of a human skin sample obtained by clearing treatment without epidermis removal treatment, which is taken under a light sheet microscope, using anti-CD31 antibody as primary antibody and AlexaFluoro594-labeled anti-sheep IgG antibody as secondary antibody. It can be understood that the structure of the region directly under the epidermis cannot be visualized.

FIG. 2(A) is a photograph of a transparent human skin sample section obtained by clearing treatment without epidermis removal treatment. An opaque layer remains on the epidermal side. FIG. 2(B) is a photograph of a transparent human skin sample section obtained by clearing treatment followed by epidermis removal treatment.

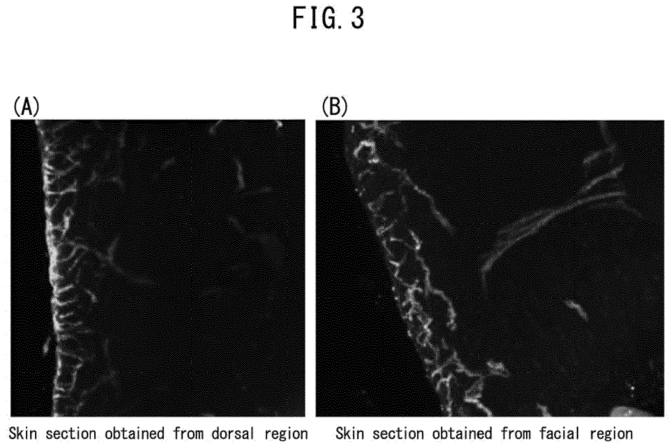

FIG. 3 is a set of three-dimensional images of human skin samples obtained by epidermis removal treatment followed by clearing treatment, which are taken under a light sheet microscope, using anti-CD31 antibody as primary antibody and AlexaFluoro594-labeled anti-sheep IgG antibody as secondary antibody. The structure of the capillaries directly under the epidermis was visualized. FIG. 3(A) is an image of a skin section obtained from a human dorsal region, and FIG. 3(B) is an image of a skin section obtained from a human facial region. In the dorsal region the capillaries have a loop structure, whereas in the facial region the capillaries have a random structure.

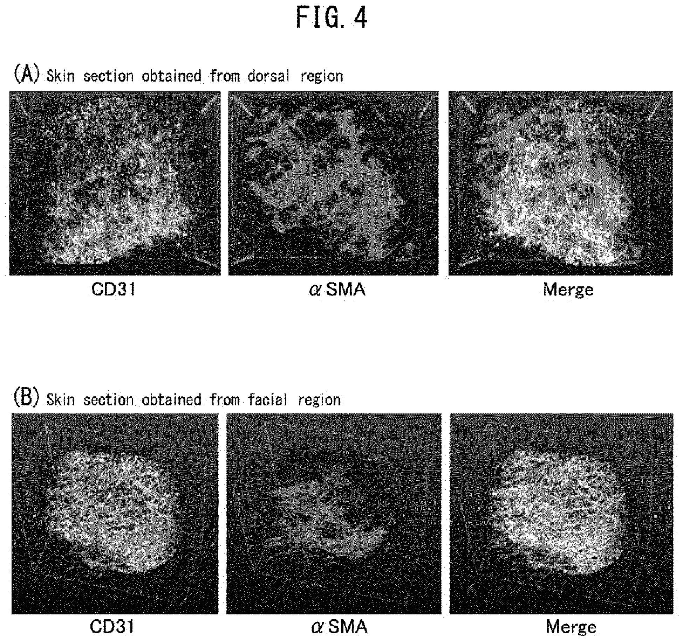

FIG. 4 is a set of three-dimensional images of human skin samples obtained by epidermis removal treatment followed by clearing treatment, which are taken under a light sheet microscope, using anti-CD31 antibody and Cy3-labeled anti-aSMA antibody as primary antibodies and AlexaFluoro488-labeled anti-sheep IgG antibody as a secondary antibody. FIG. 4(A) is a set of images of skin sections obtained from a human dorsal region, and FIG. 4(B) is a set of images of skin sections obtained from a human facial region. $\alpha$-SMA is a protein expressed in unstriated muscle, and CD31 is a protein expressed in vascular endothelial cells. FIG. 4A and B show unstriated muscle surrounding vascular endothelial cells, the structure of the capillaries differing in the skin of the dorsal and facial regions. In the skin of the dorsal region, capillaries usually appear dotted when viewing the capillaries from above, while capillaries of facial skin appear as a network even when viewed from above. That is, it is thought that loop-shaped capillaries extend upward in dorsal skin, whereas capillaries in facial skin differ by spreading out laterally.

FIG. 5 is a bar graph showing transparency for human skin samples subjected to epidermis removal treatment and cleared using a clearing treatment method.

FIG. 6A is a set of photographs of human skin samples subjected to epidermis removal treatment and cleared using a clearing treatment method. FIG. 6B is a bar graph showing haze ratios for skin samples cleared by different clearing treatment methods.

FIG. 7 is set of images of skin samples with eye corner skin, subcutaneous fatty tissue and annular muscle combined, each co-labeled with CD31, and LYVE1, perilipin or dystrophin.

FIG. 8 is a set of images of epidermis-removed skin samples from cheeks, eye corners and dorsal regions of young subject groups and aged subject groups, with the blood vessels visualized using CD31 antibody.

FIG. 9 is a set of bar graphs comparing measured volumes, diameters and branchings of visualized blood vessels, for a young subject group and an older subject group.

DESCRIPTION OF EMBODIMENTS

The present invention relates to a transparent skin sample wherein the epidermis is not accompanied with the sample, and its antigenicity is maintained, and the transparent skin sample allowing observation of subepidermal tissue under a light sheet microscope.

According to the invention, the skin sample may be a skin sample obtained from any animal species, and may even be cultured skin tissue that has been cultured using a three-dimensional culture technique. Animal species include any mammal, for example but not limited to a human, pig, horse, cow, mouse, rat, rabbit, hamster, monkey or chimpanzee. From the viewpoint of cosmetic usefulness, the skin sample is preferably obtained from a human. The site from which the skin sample is obtained may be any site, such as the face, arm, abdominal region or gluteal region. In addition, from the viewpoint of analyzing the structures of skin regions with skin trouble such as loss of skin clarity, skin roughening, blemishes, wrinkles or dermatitis, skin samples may be taken from skin regions with skin trouble.

The "transparency" may be any degree of transparency that allows observation under a light sheet microscope. For the viewpoint of allowing observation under a light sheet microscope, the transparency may be such as to allow permeation of light rays with a wavelength of 380 nm to 780 nm, and preferably light rays with a wavelength of 450 nm to 750 nm and more preferably a wavelength of 490 nm to 650 nm. A skin sample that is "transparent allowing observation of subepidermal tissue under a light sheet microscope" is not intended to be a sample to be furnished solely for a light sheet microscope, and so long as the skin sample has such transparency, it may also be observed under a common fluorescent microscope or confocal microscope. The transparency may be determined using any index, and for example, the parallel light transmittance represented by the following formula may be used:

[Formula 1]

$$T_p = (T_t - (1 - s_1) \times \alpha) / s_1 - T_d / s_1$$

{wherein:

Td is the diffuse transmittance,
Tt is the total light transmittance,
s1 is the area ratio of the skin tissue,
$\alpha$ is the total light transmittance of the cover glass (0.77), and
Tp is the parallel light transmittance of the skin tissue}. When expressed as parallel light transmittance, it is 10% to 100%. The diffuse transmittance, total light transmittance and parallel light transmittance may be calculated using a Haze meter. More specifically, it can be determined using an HR-100 produced by Murakami Color Research Laboratory Co., Ltd. The lower limit for the parallel light transmittance is preferably 20% or higher and more preferably 25% or higher, from the viewpoint of observing subepidermal tissue under a light sheet microscope. From the viewpoint of using a focus method, the transparency is preferably 30% or higher. From the viewpoint of using the iDISCO method, the transparency is preferably 40% or higher and even more preferably 45% or higher. The upper limit is not particularly restricted, but is no higher than 90%, more preferably no higher than 80% and even more preferably no higher than 60% as a practically achievable numerical range.

Clearing of a skin sample may be carried out by known clearing treatment such as the clearing treatment described in NPLs 1 and 2. Clearing treatment is carried out by contacting the skin sample with a clearing agent. The clearing agent includes an organic solvent clearing agent or water-soluble clearing agent. The examples of organic solvent clearing agents include iDISCO and BABB and the examples of water-soluble clearing agents include CLARITY, CUBIC, Scale/S and FocusClear. These clearing agents can be used according to established methods (NPLs 1 and 2). Clearing treatment is usually carried out on skin samples that have been subjected to fixing treatment and antibody labeling treatment, but labeling may also be carried out during the clearing treatment.

When the iDISCO method is used as clearing treatment, the method of producing the transparent skin sample of the invention comprises the following steps:

a step of contacting a skin section with an enzyme solution for epidermis removal,
a step of fixing the skin section by contact with a fixing solution,
a step of contact with a sodium azide-containing solution,
a step of contact with a methanol solution,
a step of contact with a dichloromethane solution, and
a step of fixing with a benzyl ether solution.

A labeling step of contact with an antibody-containing solution may also be included. The labeling step may be carried out after the step of contact with a sodium azide-containing solution.

When the BABB method is used as the clearing treatment, the method of producing the transparent skin sample of the invention comprises the following steps:

a step of contacting a skin section with an enzyme solution for epidermis removal,

a step of fixing the skin section by contact with a methanol solution, and
a step of contact with a BABB solution (a mixed solution of benzyl alcohol and benzyl benzoate).

A labeling step of contact with an antibody-containing solution may also be included. The labeling step may also be carried out after the step of contact with a methanol solution.

The methanol solution may be sequentially exchanged from a dilute methanol solution (for example, 33%) to 100% methanol.

When the CUBIC method is used as the clearing treatment, the method of producing the transparent skin sample of the invention comprises the following steps:

a step of contacting a skin section with an enzyme solution for epidermis removal,
a step of fixing the skin section by contact with a fixing solution,
a step of contact with a CUBIC-1 solution containing amino alcohol, a surfactant and urea, and
a step of contact with a CUBIC-2 solution containing amino alcohol, a surfactant and a saccharide.

A labeling step of contact with an antibody-containing solution may also be included. The labeling step may be carried out after the step of contact with a CUBIC-1 solution. The amino alcohol used in the CUBIC-1 includes N,N,N',N'-tetrakis(2-hydroxypropyl)ethylenediamine, and the surfactant includes TritonX-100. The amino alcohol used in the CUBIC-2 solution includes 2,2',2"-nitrilotriethanol, the surfactant includes TritonX-100, and the saccharide includes sucrose.

When the Scale/S method or FocusClear method is used as the clearing treatment, the method of producing the transparent skin sample of the invention comprises the following steps:

a step of contacting the skin section with an enzyme solution for epidermis removal,
a step of fixing the skin section by contact with a fixing solution, and
a step of contact with a Scale/S solution or FocusClear solution.

A labeling step of contact with an antibody-containing solution may also be included. The labeling step may be carried out after the fixing step.

A skin sample which is not accompanied with an epidermis is a sample wherein the epidermis has been removed by physical, chemical or enzymatic treatment. The epidermis is present on the outermost layer of the skin and is composed of the stratum corneum, granular layer, stratum spinosum and basal lamina in order from the outermost layer, being separated from the dermis by the epidermal basal membrane. While it is not intended to be limited by theory, the epidermis includes melanocytes that make it difficult to clear and a stratum corneum with a high refractive index, and thus clearing by clearing treatment is difficult. Therefore, it turns out that epidermis-containing transparent skin samples do not allow observation of the structure directly under the epidermis (Fig. 1). A skin sample not accompanied with the epidermis can be one that substantially lacks the epidermis. Substantially lacking the epidermis may include some epidermal cells so long as it is still possible to observe the subepidermal tissue under a light sheet microscope as the object of the invention, and epidermis that is permeable to the laser of a light sheet microscope may also be included in the skin sample. Furthermore, the skin sample may include with the skin any subcutaneous fatty tissue or muscle tissue present in deeper sections than the dermis layer.

The physical treatment for epidermis removal includes heat treatment or releasing treatment using a scalpel or forceps, the epidermal region being removed either directly visually or under a microscope. Enzymatic treatment includes treatment with a protease. The protease may include a nonspecific enzyme or specific enzyme, and Dispase, trypsin or the like may be used for the purpose of separating epidermal cells. Since the boundary between the epidermis and dermis has a complex structure known as the "papillary layer", enzymatic treatment is preferred from the viewpoint of efficient removal of the epidermis. When the epidermis is removed by enzymatic treatment, the skin sample with the epidermis removed can be obtained while maintaining the papillary structure. Moreover, since antigenicity of dermal proteins is lost with physical treatment such as heat treatment, enzymatic treatment is more suitable from the standpoint of maintaining antigenicity.

A skin sample with antigenicity maintained is a skin sample wherein corresponding antibodies can specifically bind to various protein epitope sites in the skin sample. A skin sample with antigenicity maintained does not necessarily imply that all of the antigenicity is maintained. In the field of immunostaining, some antigenicity is usually lost during treatment such as fixing treatment, and even antibodies having specific bindability for purified proteins or epitopes do not always specifically bind in fixed samples. According to the invention, therefore, "having antigenicity maintained" means that at least one or more antibodies have the property of binding with specificity to an epitope. When clearing treatment is carried out after antibody treatment, the transparent skin sample will include labeled antibodies bound to their target antigens. Without any intention to be limited to the following, preferably antigenicity is maintained for one or more proteins, for example, selected from the group consisting of CD31, LYVE1, perilipin and dystrophin. From the viewpoint of visualization of vessels directly under the skin, preferably antigenicity is maintained for CD31 and LYVE1.

Visualization can be achieved by supplying the transparent skin sample to a light sheet microscope and allowing specific proteins in the skin sample to be recognized by antibody. The desired structure in the skin can also be observed by using antibodies for proteins specifically expressed in the desired structure in the skin. Skin structure includes, without intention to be limited thereto, extracellular matrix, lymphatic vessels, veins, arteries, capillaries, nerves, sweat glands, sebaceous glands, and cell components such as mast cells, plasmocytes, fibroblasts, Langerhans cells, Merkel cells, vascular endothelial cells, lymphatic endothelial cells, nerve cells and sweat gland cells. For visualization of blood vessels, for example, antibodies for proteins specifically expressed in vascular cells, such as CD31, vWF and CD34, may be used. For visualization of extracellular matrix, antibodies for collagen, elastin, $\alpha$SMA and fibronectin, etc., may be used. For visualization of nerves, antibodies for PGP9.5 etc., may be used. For visualization of lymphatic vessels, antibodies for LYVE-1 and podoplanin etc., may be used. These antibodies may be used alone or in combinations.

The antibodies used for observation of a transparent skin sample may be antibodies obtained from any desired animal species, or antibodies produced by a genetic engineering method such as the phage display method. The animal species includes a mouse, human, rat, rabbit, goat, camel, donkey or the like, and antibodies may be obtained by introducing an antigen into these animals. The antibodies may be monoclonal antibodies or polyclonal antibodies. They may also be chimeric antibodies that are a combination of these antibodies. Bindable antibody fragments may also be used instead of antibodies. Examples of antibody fragments include Fab fragment, Fv fragment, $F(ab')_2$ fragment, Fab' fragment and scFv.

The antibody itself that directly binds antigen may be labeled to allow observation under a light sheet microscope, or secondary antibodies that bind to the antibody directly bound to the antigen, or further antibodies may be labeled. The added label is preferably any fluorescent labeling used for fluorescent microscopes, for examples, including any fluorescent labeling such as rhodamine, fluorescein, Cy dye or Alexa. Multiple antigen can be simultaneously and continuously visualized by using antibodies that respectively bind to multiple antigens, and differentially labelled secondary antibodies which bind distinctly to each antibody.

In addition to labeling with antibodies, or instead of labeling with antibodies, the cells in the transparent skin sample may be nuclear stained, or fluorescent proteins may be expressed. The nuclear stain reagent used may be a publicly known fluorescent reagent, examples of which include DAPI, propidium iodide (PI) and Hoechst 33342. The nuclear staining or antibody fluorescent labeling is preferably selected so as to have a fluorescent wavelength allowing their separate identification. Fluorescent proteins such as GFP can be utilized by creating a transgenic animal having the GFP or YFP gene introduced downstream from a desired promoter, or by locally expressing a vector in the animal.

According to another aspect of the invention, it relates to a method of producing a transparent skin sample from a skin section, or a method of treating the skin section. The skin section used may be a previously obtained skin section. Such a method may include the following steps in any desired order:

contacting the skin section with an enzyme solution for epidermis removal to remove the epidermis,
fixing the skin section by contacting with a fixing solution, and
clearing the skin section by contact with a clearing reagent. The epidermis removal step, fixing step and clearing step are preferably carried out in this order. The method may further include, after the fixing step, a labeling step in which the skin section is contacted with a labeled antibody solution. A washing step may also be included before and after each step. According to yet another aspect of the invention, the invention relates to a transparent skin sample produced by a method of producing a transparent skin sample from a skin section.

The epidermis removal step is carried out, for example, by incubating in an enzyme solution for epidermis removal for several hours to several days at room temperature, or with heating or cooling. From the viewpoint of accelerating the enzyme reaction, the temperature is preferably near 37°C, such as 33°C to 40°C. From the viewpoint of preventing protein denaturation, on the other hand, the incubation is preferably carried out with cooling, for example, the incubation preferably being at 0°C to 5°C and more preferably 4°C to 5°C. From the viewpoint of proper removal of the epidermis, it is preferably incubation for 1 hour to 2 days and more preferably incubation for 3 hours to 12 hours with cooling. For removal of the epidermis alone, preferably the epidermal side of the skin section is contacted with a support such as gauze that has been wetted with enzyme solution, and incubated. More properly, incubation is carried out with the epidermis of the skin section placed facing downward on a water-absorbing support such as gauze that has been wetted with enzyme solution. The incubating conditions may differ depending on the site from which the sample has been obtained, and they may be changed depending on the state of the sample site. With rough skin, for example, in which the skin barrier function is reduced and permeation of the enzyme solution is therefore more rapid, weak incubating conditions such as a short time and low temperature may be selected. After contact with the enzyme solution, forceps or the like are used to separate the epidermis from the dermis, thereby removing the epidermis.

The fixing step may be carried out by a method commonly used in the field of immunostaining. Paraformaldehyde, methanol or the like can be used as a fixing solution. As an example, incubation is carried out for several minutes to several days with the skin section immersed in a 4% paraformaldehyde solution, either at room temperature or with cooling. Since a fixed sample is less affected by enzymatic treatment, the fixing step is preferably carried out after the epidermis removal step.

The labeling step may be carried out by a method commonly used in the field of immunostaining. For example, the fixed sample may be incubated in a primary antibody solution of an antibody for a target antigen, and washed, and then incubated in a solution of a labeled secondary antibody against the primary antibody. The antibody dilution ratio, incubation time and temperature may be appropriately selected for the antibody used. In order to avoid fading, incubation with the labeled secondary antibody solution is preferably carried out in a dark environment. The treatment and storage after the labeling step are preferably carried out entirely in a dark environment.

The clearing step is carried out by incubation of the sample in a solution of a known clearing reagent. Examples of clearing steps include treatment by the iDISCO method (iDISCO: A Simple, RapidMethod to Immunolabel Large Tissue Samples for Volume Imaging. Cell 159, 896910, November 6, 2014), CUBIC method, Scale method or FocusClear method. The clearing reagent and incubation time may be appropriately selected to obtain a sufficiently transparent sample.

The transparent skin sample that has been prepared after the labeling step is subjected to observation under a light sheet microscope or fluorescent microscope. Observation under a light sheet microscope or fluorescent microscope may be carried out by a method commonly employed for such microscopes. For example, by selecting an incident beam suited for the attached label and selecting a filter suited for the excitation light, it is possible to observe the excitation light from the label.

The transparent skin sample of the invention may be used to observe the internal microstructure of skin, and is designed to accumulate knowledge regarding the internal structure of the skin. The internal structure of skin in skin regions with skin troubles such as skin roughening, blemishes, wrinkles, liver spots or pimples can be precisely observed to aid in understanding their causes and developing ameliorating and curing methods. Although the present invention requires the use of skin sections obtained in an invasive manner, methods of observation of such sections under light sheet microscopes or fluorescent microscopes are incomparably superior to the currently developed noninvasive methods of observation of the internal structure of skin, and their visualized structures are also more distinct. It is therefore possible to accumulate data and knowledge regarding the internal structure of skin, before observation of the internal structure of skin by noninvasive methods that are expected to be utilized in the future.

All of the publications mentioned throughout the present specification are incorporated herein in their entirety by reference.

The examples of the invention described below are intended to serve merely as illustration and do not limit the technical scope of the invention. The technical scope of the invention is limited solely by the description in the Claims. Modifications of the invention, such as additions, deletions or substitutions to the constituent features of the invention, are possible so long as the gist of the invention is maintained.

EXAMPLES

Enzymatic treatment step

Dispase (Roche) (38 U/vial) was dissolved in 38 ml of Milli-Q water. A Kimwipe was seated on a dish, and immersed in the Dispase. The epidermal side of a 0.5 mm-square human skin section was immersed in the Dispase facing downward and incubated overnight at 4°C. On the following day, the stratum corneum was removed using forceps.

Clearing treatment using CUBIC

A 5 mm-cubic human skin section was immersed in 4% paraformaldehyde (PFA), rotated with a Rotator RT-50 (TAITEC), incubated overnight at 4°C, and fixed. The fixed skin section sample was permeated with a CUBIC-1 solution for 1 week. After the CUBIC-1 treatment the sample was washed 3 times with PBS and rotated overnight at 4°C in 20% sucrose solution. Then, it was frozen in an O.C.T compound (Sakura Finetech). After thawing, it was washed 3 times with PBS and then permeated in primary antibody solution at 37°C for 3 days. After washing 3 times with PBST (PBS + 0.1% triton), it was permeated overnight in secondary antibody solution at 37°C. After washing 3 times with PBST (PBS + 0.1% triton), it was immersed overnight in 20% sucrose, placed in a CUBIC-2 solution and incubated at 4°C for 1 week. The compositions of the CUBIC-1 and CUBIC-2 were as described in Susaki et al., Cell. 2014 Apr 24; 157(3): 726-39. Specifically, the CUBIC-1 solution was a solution containing N,N,N',N'-tetrakis(2-hydroxypropyl)ethylenediamine, TritonX-100 and urea, and the CUBIC-2 solution was a solution containing 2,2',2"-nitrilotriethanol, TritonX-100 and sucrose.

Clearing treatment using Scale or FocusClear

A 5 mm-cubic human skin section was immersed in 4% paraformaldehyde (PFA) and fixed at 4°C. After fixing, it was washed 3 times with PBS, permeated with primary antibody at 4°C for 7 days, washed for 3 days with PBST and permeated with secondary antibody at 4°C for 7 days. After washing with PBST, it was permeated with Focus Clear (vendor: Cedarlane) or Scale (vendor: Olympus Corp.) at 4°C for 7 to 14 days.

Clearing treatment using iDISCO

A 5 mm-cubic skin sample was fixed with 4% PFA. It was then washed 3 times with PBS. After shaking in a PBS solution containing 5% Triton and 2.5% Tween20, it was washed 3 times with PBS. Then, it was shaken in Perm Block Solution (50 ml of PBS solution containing 0.5 g BSA, 50 $\mu$l Tween20, 300 $\mu$l 5% sodium azide). Next, the sample was shaken for 3 days in a solution containing primary antibody at 37°C, and washed for 3 days with PBST (0.1% Tween20). It was then shaken for 3 days in a solution containing secondary antibody at 37°C, and then washed for 3 days with PBST (0.1% Tween20). Next, it was shaken for 3 hours with 50% MeOH, for 3 hours with 70% MeOH and overnight with 100% MeOH. Finally, it was shaken for 15 minutes $\times$ 2 with dichloromethane (Sigma) and allowed to stand overnight in dibenzyl ether (Sigma).

A skin section obtained from the facial region and a skin section obtained from the dorsal region were used as skin samples. A skin sample with the epidermis removed were prepared by carrying out enzymatic treatment, and a skin sample with the epidermis remaining were prepared by not carrying out enzymatic treatment. The skin samples were subjected to clearing treatment by the iDISCO method. Fig. 2 shows a sample following clearing treatment. Anti-CD31 sheep antibody (vendor: R&D Systems) diluted 100 fold with PBS was used as a primary antibody, and AlexaFluoro594-labeled anti-sheep IgG antibody (vendor: Invitrogen) diluted 200-fold with PBS was used as a secondary antibody. A PBS-diluted Cy3-labeled anti-aSMA antibody (vendor: Sigma) was used as co-staining. The stained skin sample was observed under a light sheet microscope (manufacturer: Carl Zeiss). A photograph of a CD31-visualized skin sample with the epidermis remaining is shown in Fig. 1. Photographs of CD31-visualized epidermis-removed skin samples (a skin section obtained from the dorsal region and a skin section obtained from the facial region) are shown in Fig. 3(A) and Fig. 3(B). Also, photographs of CD31- and $\alpha$SMA-visualized and 3-dimensional structure-formed epidermis-removed skin samples (skin sections obtained from the dorsal region and skin slices obtained from the facial region) are shown in Fig. 4(A) and Fig. 4(B).

Transparency measurement method

Stratum corneum-removed skin tissues were respectively subjected to CUBIC treatment, FocusClear treatment, Scale treatment and iDISCO treatment. Measurement of the transparency of the clear-treated skin tissue was carried out using a modification of the method described in Tainaka et al., Cell. 2014 Nov 6; 159(4): 911-24. The Td: diffuse transmittance and Tt: total light transmittance of the human skin tissue were measured using a haze meter (company name: product No.). Since skin tissue did not cover the entire test piece, correction was made to area ratio (s1), and the parallel light transmittance(Tp) of the skin tissue was calculated. Specifically, calculation was performed by the following formula:

$$Tp = (Tt - (1 - s1) \times \alpha)/s1 - Td/s1$$

{wherein

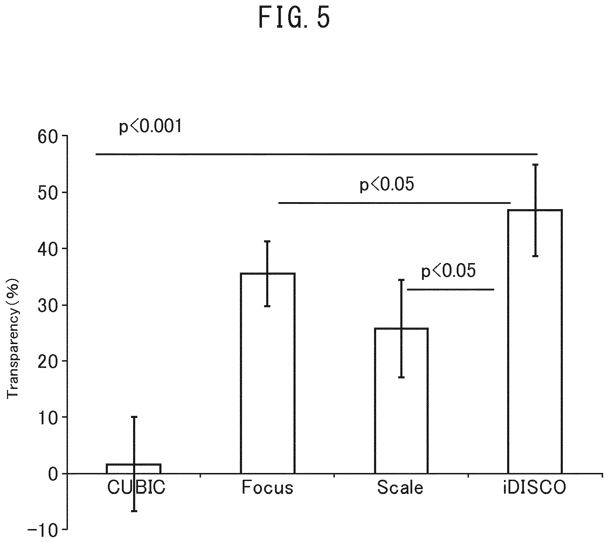

Td is the diffuse transmittance,
Tt is the total light transmittance,
s1 is the area ratio of the skin tissue,
$\alpha$ is the total light transmittance of the cover glass (0.77), and
Tp is the parallel light transmittance of the skin tissue}. The results are shown in Fig. 5.

The diffuse transmittance was also divided by the total light transmittance to calculate the haze (%).

Human skin samples

Human skin samples were obtained from ILSBIO LLC (Chestertown, MD) or (Gakugeidai-Nishiguchi Clinic). All of the skin samples from facial or dorsal regions of Asian persons which were obtained from ILSBIO, were obtained based on U.S. and International Ethical Guidelines. The ILSBIO protocol is approved by the Health and Human Services registered Institutional Review Board (IRB). Informed consent was obtained prior to sample collection.

Skin samples including the skin, annular muscle and subcutaneous fatty tissue were also obtained from the eye corners of Japanese males and females at Gakugeidai Nishiguchi Clinic. The subjects were confirmed to be free of atopic dermatitis or acne. All of the samples were quick-frozen and provided for histological analysis. All of the methods including human subjects were those approved by the Clinical Trial Review Committee at the Shiseido Global Innovation Center, and informed consent was obtained in writing from all of the subjects.

The obtained skin samples were subjected to the enzymatic treatment step described above, and the stratum corneum was removed. The stratum corneum-removed skin samples were fixed by immersion in 4% paraformaldehyde (PFA).

Clearing

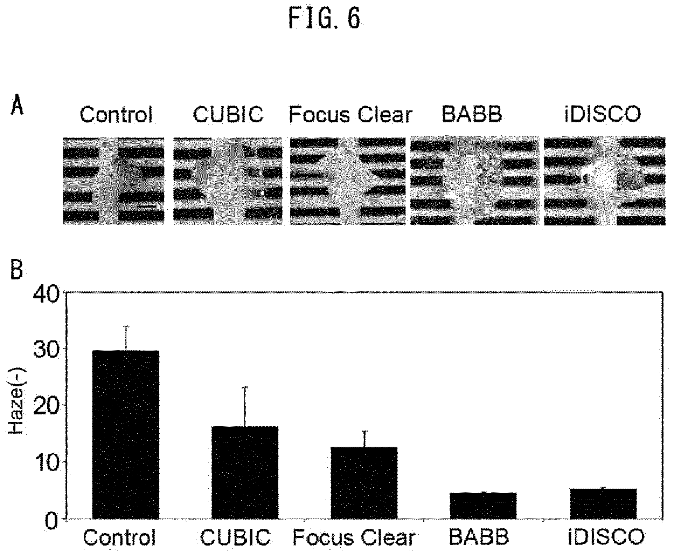

The fixed facial region skin samples were supplied to the Cubic, Focus Clear, BAAB and iDISCO methods for clearing. The cleared skin samples were photographed (Fig. 6A). The light transmittance and haze (%) of each of the cleared skin samples were measured in the manner described above. The results are shown in Fig. 6B.

Immunolabeling

Multiple labeling of skin, annular muscle and subcutaneous fatty tissue

The skin samples including skin, annular muscle and subcutaneous fatty tissue that had been fixed with PFA solution were washed with PBS and subjected to clearing treatment with 0.5% TritonX-100 in PBS, and subsequently incubated with 1% Triton X-100/0.5% Tween-20 in PBS. After incubation for 3 days with a blocking solution, each sample was incubated with primary antibody in a blocking solution at 37°C for 3 days. The primary antibody used was anti-polyclonal sheep antibody for CD31 (R&D Systems, Minneapolis, MN), polyclonal rabbit antibody for LYVE-1 (Angiobio, San Diego, CA), polyclonal guinea pig antibody for perilipin (Progen, Heidelberg, Germany) or polyclonal rabbit antibody for dystrophin (Santa Cruz Biotechnology, Dallas, Texas). Skin samples co-labeled with anti-CD31 antibody and anti-LYVE1 antibody, skin samples co-labeled with anti-CD31 antibody and anti-perilipin antibody and skin samples co-labeled with anti-CD31 antibody and anti-dystrophin antibody were obtained. The co-labeled skin samples were rinsed for 2 days with PBS-T, and then multi-labeled by incubation at 37°C for 3 days using blocking solution-diluted AlexaFluoro594-labeled anti-sheep IgG antibody (vendor: Invitrogen) and AlexaFluoro488-labeled anti-rabbit IgG antibody or AlexaFluoro488-labeled anti-guinea pig IgG antibody as secondary antibodies.

Single labeling of CD31 of cheek, eye corner and dorsal region skin samples

The cheek, eye corner and dorsal region skin samples that had been fixed with PFA solution were washed with PBS and subjected to clearing treatment with 0.5% TritonX-100 in PBS, and subsequently incubated with 1% Triton X-100/0.5% Tween-20 in PBS. After incubating with a blocking solution for 3 days, each sample was incubated with a primary antibody in a blocking solution at 37°C for 3 days. Polyclonal sheep antibody for CD31 (R&D systems, Minneapolis, MN) was used as the primary antibody. The samples were rinsed with PBS-T for 2 days and then single-labeled by incubation for 3 days at 37°C using blocking solution-diluted AlexaFluoro594-labeled anti-sheep IgG antibody (vendor: Invitrogen) as the secondary antibody.

The immunolabeled samples were subjected to the iDISCO method and cleared. The cleared skin samples were subjected to a microscope and for imaging analysis.

Microscope and imaging analysis

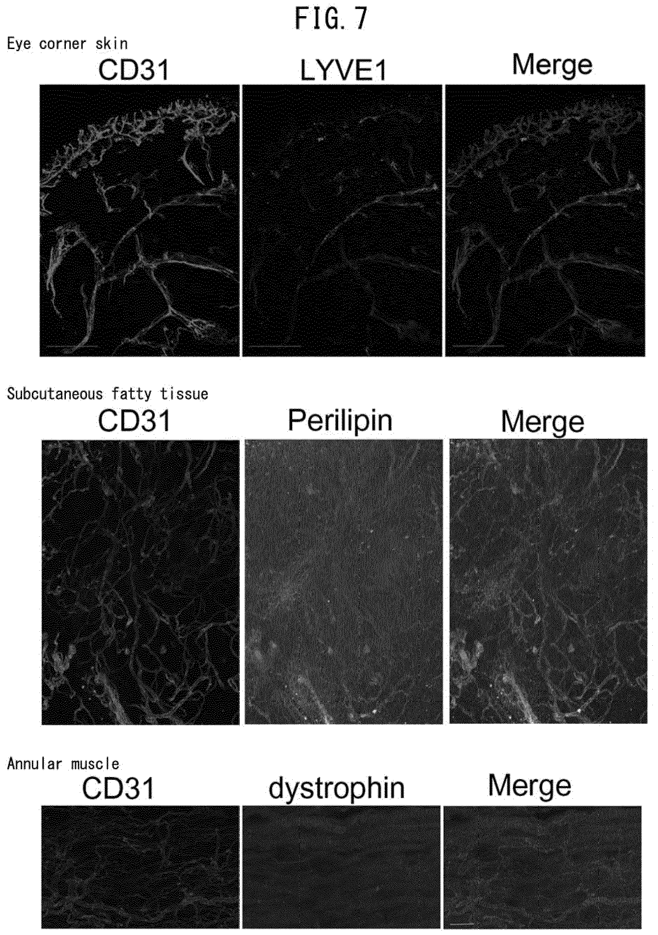

Images were obtained for the immunolabeled skin samples using a light sheet fluorescent microscope (Lightsheet microscopy Z.1, Carl Zeiss, Germany). Maximum projection was achieved using software Zen (Carl Zeiss). Imaris software (Bitplane, Concord, MA) was used for 3D imaging. CD31, LYVE1, perilipin and dystrophin were respectively visualized in the skin regions for the skin samples co-labeled with anti-CD31 antibody and anti-LYVEI antibody, the subcutaneous tissue regions for the skin samples co-labeled with anti-CD31 antibody and anti-perilipin antibody, and the muscle layer regions for the skin samples co-labeled with anti-CD31 antibody and anti-dystrophin antibody, and then three-dimensional images were obtained (Fig. 7).

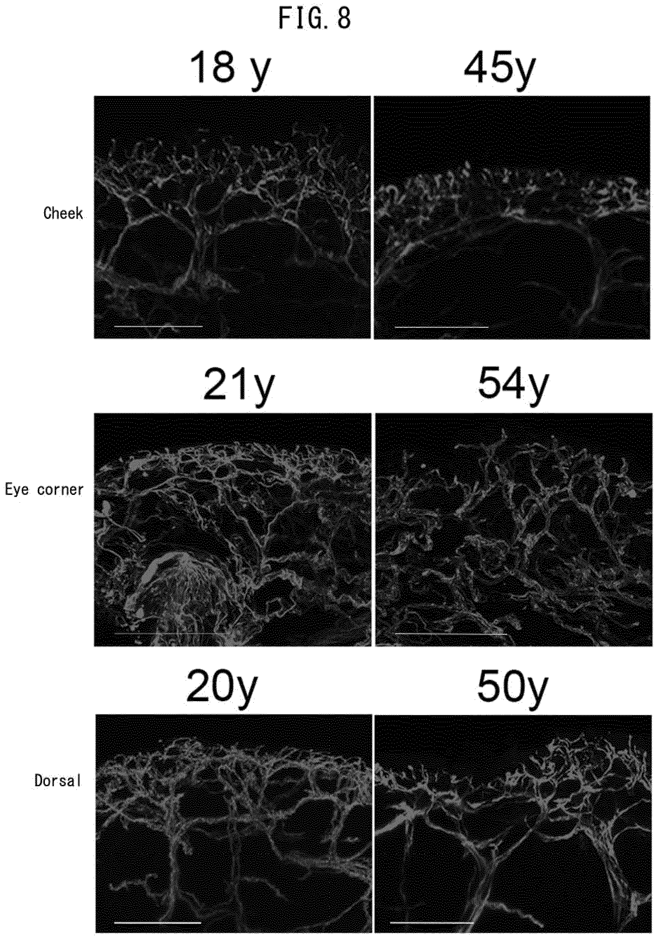

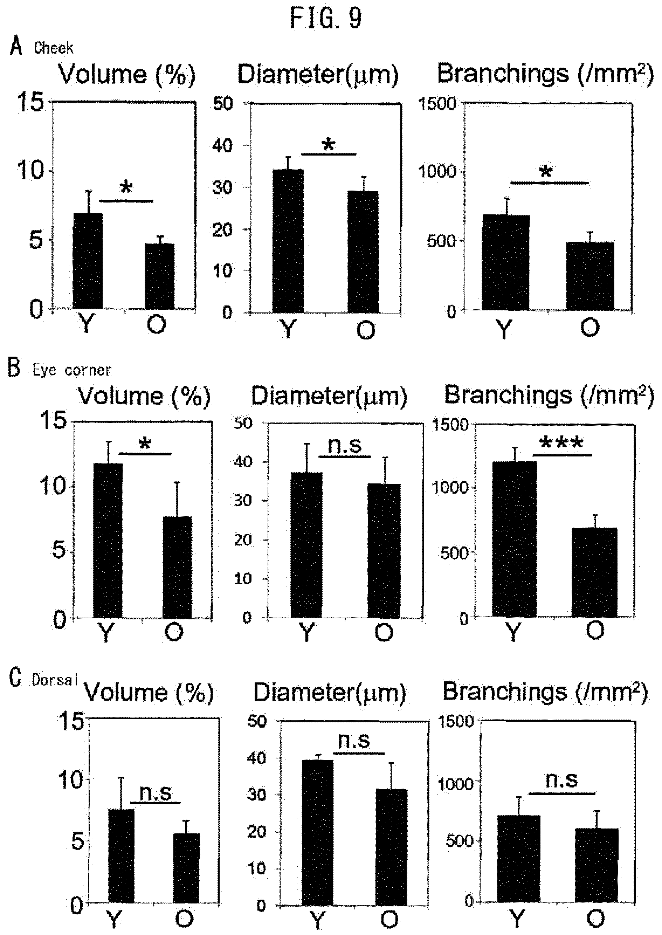

The cheek, eye corner and dorsal region skin samples were divided into 2 age groups: a "young group" (for cheek skin, average age = 20.8 ±4.8 years, age range = 18-29 years, n = 4; for eye corner skin, average age = 20.5 ±5.4 years, age range = 12-27 years, n = 4; for dorsal region skin, average age = 19.3 ±6.4 years, age range = 18-20 years, n = 3)", and an "older group" (for cheek skin, average age = 46.3 ±2.2 years, age range = 45-50 years; for eye corner skin, average age = 51.3 ±9.7 years, age range = 35-61 years, n = 4:; for dorsal region skin, average age = 44.3 ±4.1 years, age range = 39-49 years, n = 3). The blood vessels in the cheek, eye corner and dorsal region skin subjects were visualized by labeling using anti-CD31 antibody as described above, and photographed (Fig. 8). Morphological three-dimensional analysis was carried out as previously reported (NPL 4: Bise R, Sato I, Kajiya K, et al. (2016), 3D Structure Modeling of Dense Capillaries by Multi-Objects Tracking. Proceedings of the IEEE Conference on Computer Vision and Pattern Recognition Workshops: 29-37), and blood vessel volumes and sizes and capillary branchings were measured from the images (Fig. 9).

Statistical analysis

All of the statistics shown are mean ±SD. Statistical analysis was performed using ANOVA, followed by a post-hoc statistical test. The Tukey statistical test and subsequently the Bartlett test were used for evaluation of the statistically significant difference in comparing the control group and multiple groups. A significant difference was considered to be $P < 0.05$.

**Claims**

1.  A transparent skin sample that allows observation of subepidermal tissue under a light sheet microscope, wherein

    the transparent skin sample is not accompanied with a light-impermeable epidermis, and
    the antigenicity is maintained.

2.  The transparent skin sample according to claim 1,
    wherein the parallel light transmittance of the transparent skin sample is 10% to 100%, when the transparency is measured according to the following formula:

    [Formula 1]

    $$T p = (T t - (1 - s 1) \times \alpha) / s 1 - T d / s 1$$

    {wherein:

    Td is the diffuse transmittance,
    Tt is the total light transmittance,
    s1 is the area ratio of the skin tissue,
    $\alpha$ is the total light transmittance of the cover glass (0.77), and
    Tp is the parallel light transmittance of the skin tissue}.

3.  The transparent skin sample according to claim 1 or 2, wherein the transparent skin sample is from a human.

4.  A method of producing a transparent skin sample from an obtained skin section, comprising:

    contacting the skin section with an enzyme solution for epidermis removal,
    fixing the skin section by contacting the skin section with a fixing solution, and
    contacting the skin section with a clearing reagent.

5.  The method according to claim 4, further comprising a step of contacting the skin section with a labeled antibody solution after the fixing step.

6.  The method according to claim 4 or 5, wherein the skin sample is from a human.

7. The method according to any one of claims 4 to 6, wherein the enzyme solution is Dispase solution.

8. The method according to any one of claims 4 to 7, wherein the fixing solution is a paraformaldehyde solution.

9. The method according to any one of claims 4 to 8, wherein the clearing reagent comprises an organic solvent-based clearing reagent.

10. The method according to claim 9, wherein the organic solvent-based clearing reagent includes iDISCO.

11. A transparent skin sample produced by the method according to any one of claims 4 to 10, wherein

the transparent skin sample is not accompanied with the epidermis,
the parallel light transmittance of the transparent skin sample is 10% to 100%, when the transparency is measured in accordance with the following formula:

[Formula 2]

$$T_p = (T_t - (1 - s1) \times \alpha) / s1 - T_d / s1$$

{wherein:

$T_d$ is the diffuse transmittance,
$T_t$ is the total light transmittance,
s1 is the area ratio of the skin tissue,
$\alpha$ is the total light transmittance of the cover glass (0.77), and
$T_p$ is the parallel light transmittance of the skin tissue}.

12. The transparent skin sample according to claim 11, wherein the transparent skin sample allows observation of subepidermal tissue under a light sheet microscope.

13. A method of treating a skin section, comprising

contacting the skin section with an enzyme solution for epidermis removal,
fixing the skin section by contact the skin section with a fixing solution, and
contacting the skin section with a clearing reagent,

in any desired order.

14. The method of treating a skin section according to claim 13, further comprising a step of contacting the skin section with a labeled antibody solution.

15. The method according to claim 13 or 14, wherein the skin sample is from a human.

16. The method according to any one of claims 13 to 15, wherein the enzyme solution is Dispase solution.

17. The method according to any one of claims 13 to 16, wherein the fixing solution is a paraformaldehyde solution.

18. The method according to any one of claims 13 to 17, wherein the clearing reagent is an organic solvent-based clearing reagent.

19. The method according to claim 18, wherein the organic solvent-based clearing reagent is iDISCO.

20. A method of observing a skin sample that has been treated according to the method of treating a skin section according to claim 14, under a light sheet microscope.

# FIG. 1

# FIG. 2

(A)

(B)

# FIG. 3

(A)

Skin section obtained from dorsal region

(B)

Skin section obtained from facial region

# FIG. 4

(A) Skin section obtained from dorsal region

CD31        $\alpha$ SMA        Merge

(B) Skin section obtained from facial region

CD31        $\alpha$ SMA        Merge

# FIG. 5

## FIG. 6

## FIG. 7

Eye corner skin

CD31　　　　　LYVE1　　　　　Merge

Subcutaneous fatty tissue

CD31　　　　　Perilipin　　　　　Merge

Annular muscle

CD31　　　　　dystrophin　　　　　Merge

## FIG. 8

18 y      45y

Cheek

21y      54y

Eye corner

20y      50y

Dorsal

# FIG. 9

A Cheek

B Eye corner

C Dorsal

**INTERNATIONAL SEARCH REPORT**

| International application No. |
|---|
| PCT/JP2017/029126 |

| A. CLASSIFICATION OF SUBJECT MATTER |
|---|
| C12N5/071(2010.01)i, G01N1/28(2006.01)i, G01N1/30(2006.01)i |

According to International Patent Classification (IPC) or to both national classification and IPC

| B. FIELDS SEARCHED |
|---|
| Minimum documentation searched (classification system followed by classification symbols) |
| C12N5/071, G01N1/28, G01N1/30 |

| Documentation searched other than minimum documentation to the extent that such documents are included in the fields searched |
|---|
| Jitsuyo Shinan Koho          1922–1996    Jitsuyo Shinan Toroku Koho   1996–2017 |
| Kokai Jitsuyo Shinan Koho    1971–2017    Toroku Jitsuyo Shinan Koho   1994–2017 |

| Electronic data base consulted during the international search (name of data base and, where practicable, search terms used) |
|---|
| JSTPlus/JMEDPlus/JST7580(JDreamIII), CAplus/MEDLINE/EMBASE/BIOSIS(STN), WPIDS/WPIX(STN) |

C. DOCUMENTS CONSIDERED TO BE RELEVANT

| Category* | Citation of document, with indication, where appropriate, of the relevant passages | Relevant to claim No. |
|---|---|---|
| A | Atsuyoshi OSADA et al., "Koso Shori no Umu ga Baiyo Hyohi Sheet Kiteimaku ni Oyobosu Eikyo no Kento", Regenerative Medicine special extra issue, 27 January 2014 (27.01.2014), vol.13, special extra issue, page 229, O-31-1 | 1-20 |
| A | JP 2013-048642 A (Tohoku University), 14 March 2013 (14.03.2013), & WO 2011/074208 A1 | 1-20 |
| A | JP 2014-005231 A (Riken, Japan), 16 January 2014 (16.01.2014), & US 2016/0169776 A1      & WO 2013/191274 A1 & EP 2866017 A1 | 1-20 |

| ☒ | Further documents are listed in the continuation of Box C. | ☐ | See patent family annex. |
|---|---|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 November 2017 (01.11.17) | 14 November 2017 (14.11.17) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**EP 3 498 823 A1**

| INTERNATIONAL SEARCH REPORT | International application No. |
|---|---|
| | PCT/JP2017/029126 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2016/117614 A1  (Riken Institute of Physical and Chemical Research), 28 July 2016 (28.07.2016), & CN 107209093 A | 1-20 |

Form PCT/ISA/210 (continuation of second sheet) (January 2015)

22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2014005231 A

### Non-patent literature cited in the description

- *Cell,* 2014, vol. 159 (4), 896-910
- *Nature Neuro Science,* 2015, vol. 18 (10), 1518-1529
- *Cell,* 2014, vol. 157 (3), 726-39
- *Proceedings of the IEEE Conference on Computer Vision and Pattern Recognition Workshops,* 29-37
- **SUSAKI et al.** *Cell,* 24 April 2014, vol. 157 (3), 726-39
- **TAINAKA et al.** *Cell,* 06 November 2014, vol. 159 (4), 911-24
- **BISE R ; SATO I ; KAJIYA K et al.** 3D Structure Modeling of Dense Capillaries by Multi-Objects Tracking. *Proceedings of the IEEE Conference on Computer Vision and Pattern Recognition Workshops,* 2016, 29-37